# EUROPEAN PATENT SPECIFICATION

(11) **EP 0 438 292 B1**
(45) Date of publication and mention of the grant of the patent: **16.10.1996**
(21) Application number: 91300326.5
(22) Date of filing: 17.01.1991
(51) Int. Cl.: C12P 19/34, C12Q 1/68, C12N 15/10

(54) **Method of preparing primers for polymerase chain reaction and DNA amplification using said primers**
Verfahren zur Vorbereitung von Primern für die Polymerase-Kettenreaktion und DNS-Verstärkung durch Verwendung dieser Primer
Procédé pour la préparation d'amorces pour la réaction en chaîne de polymérase à l'aide de ces amorces

(30) Priority: 17.01.1990 JP 9302/90
(43) Date of publication of application: 24.07.1991
(73) Proprietor: NIHON CHEMICAL RESEARCH KABUSHIKI KAISHA also known as JCR PHARMACEUTICALS CO., LTD, Kobe Hyogo 658 (JP)
(72) Inventor: Tanaka, Kunisuke, Sakyo-ku, Kyoto 606 (JP); Masumura, Takehiro, Nishi-ku, Kobe, Hyogo 673 (JP); Koga, Junichi, Kobe, Hyogo 673 (JP); Hirantani, Hajime, Sennan-gun, Osaka 599-02 (JP)
(74) Representative: Smart, Peter John

(56) References cited:
- EP-A- 0 200 362
- J. SAMBROOK et al.: "Molecular cloning, a laboratory manual", edition 2, 1989, vol. 1, chapter 5, Cold Spring Harbor Laboratory Press, New York, US
- NUCLEIC ACIDS RESEARCH, vol. 17, no. 14, 1989, page 5865, IRL Press; R.G.
- HIGUCHI et al.: "Production of single-stranded DNA templates by exonuclease digestion following the polymerase chain reaction"
- GENE, vol. 97, 1991, pages 1-6, Elsevier Science Publishers B.V. (Biomedical Division); V. SHYAMALA et al.: "Use of exonuclease for rapid polymerase-chain- reaction-based in vitro mutagenesis"

## Description

This invention relates to a method of preparing novel primers that can be used in the in vitro polymerase chain reaction (PCR) and to a method of DNA amplification using said primers.

Establishment of several fundamental techniques has much contributed to the progress of molecular biology. The cloning, sequencing and blotting techniques are typical examples. The recently reported PCR method (R. Saiki et al., 1985, Science, 230: 1350; K. B. Mullis and F. A. Fallona, 1987, Methods Enzymol., 155: 335~350) the application of which has already begun is becoming one of such fundamental techniques. This technique is characterized in that a DNA sequence sandwiched between a pair of chemically synthesized single-stranded DNAs (primers) can be selectively amplified in vitro. This technique has also enabled amplification of a desired DNA chain from a very small amount of template by holding a reaction mixture composed of the template DNA, NTPs, primers and a heat-resistant DNA polymerase in an automated block heater (thermal cycler) and repeating the cycle consisting of DNA annealing, polymerase-catalyzed novel DNA chain synthesis and deannealing. By applying this technique, it is now possible not only to obtain a specific region of a known DNA chain in an easy and simple manner but also to readily detect defects in chromosomal genes, for example deletions in the muscular dystrophy-related gene (J. S. Chamberlain, 1988, Nucl. Acids Res., 16: 11141~11156). However, the application of this series of techniques to the analysis of a gene having a quite unknown base sequence has been very much limited. Recently, the so-designated inverted PCR method was reported for overcoming the above drawback and analyzing chromosomal genes (T. Triglia et al., 1988, Nucl. Acids Res., 16: 8186). This method comprises ligating, using ligase, gene fragments of a chromosome prepared by cleaving with an appropriate restriction enzyme to give a number of small-sized circular DNAs and subjecting these, together with those DNAs corresponding to both ends of a known gene (e.g. a specific cDNA) as synthesized in opposite orientation to that for normal PCR, to the reaction. This method has made it possible to amplify DNA base sequences adjacent to a known chromosomal coding gene, for example a specific cDNA and to analyze their functions, for example the structures and functions of those genes that cannot be captured as cDNAs, e.g. promoters and terminators. However, this technique has some drawbacks. Firstly, chemical synthesis of primers is indispensable. Secondly, for designing the primers to be chemically synthesized, the relevant base sequence must be known. Thirdly, when the introns and cDNA fail to predict the base sequence adjacent to a cDNA coding region, said technique is not applicable to the determination of said sequence. Thus, even such state-of-the-art technique is limited in its capacity to apply the PCR method to gene analyses.

The use of an exonuclease in combination with a DNA polymerisation step is disclosed in Sambrook et al., Molecular Cloning, 2^{nd} ed., 1989, 5.84-5.86 and 5.45. The resected double-stranded fragment generated by the exonuclease, however, is never denatured and is not used in an amplification method.

The present inventors made intensive investigations and, as a result, succeeded in producing entirely new primers which are useful in the PCR. They are not chemically synthesized DNAs but are fragments having an appropriate length as prepared by digesting a DNA chain on hand (e.g. cDNA) from the 5′ terminus side using a particular enzyme; they can be used each as a primer. Then, the inventors carried out the PCR method using such a primer as mentioned above and, as a template, a plasmid with a cDNA cloned therein. As a result, it was found that the plasmid can be amplified as such with a very high efficiency. This finding led to an invention consisting in a novel technique by which a plasmid containing a cloned cDNA in question can be amplified and purified in a reaction mixture without introduction thereof into bacterial cells, whereby the transformation efficiency can be greatly increased.

The inventors then applied said primers to the amplification of a chromosomal gene. Thus, a DNA derived from a eukaryotic cell chromosome was cleaved with a restriction enzyme and a circular DNA was prepared by causing self-ligation using ligase. The PCR was carried out using any of the primers mentioned above with said circular DNA as a template. Surprisingly, it was found that, of both the chromosomal genes adjacent to the primer, which is a fragment of an available DNA chain, only those portions which are between the recognition sites for the restriction enzyme used for cleavage are amplified while no other genes are amplified at all. The invention was thus found to be very effective in analyzing a coding gene and related regulatory genes on a chromosome using a cDNA owing to the specificity and simplicity of the technique involved. Based on the findings mentioned above, the present invention has been completed.

The invention thus provides a method of amplifying DNA in vitro by the polymerase chain reaction which method comprises using, as a primer, a DNA fragment obtained by treating a double-stranded DNA fragment with an exonuclease, and a method of amplifying a chromosomal gene which comprises carrying out the inverted polymerase chain reaction using, as a primer, a DNA fragment obtained by treating a double-stranded DNA fragment with an exonuclease capable of digesting one chain of the double-stranded DNA in a manner such that mononucleotides are released therefrom in the direction from the 5′ terminus to the 3′ terminus and, as a template, a circular DNA obtained by cleaving the chromosomal gene with a restriction enzyme and subjecting the resulting fragment to self-ligation.

The present invention provides very effective means for cloning an unknown gene for the analysis thereof by applying the PCR or inverted PCR method. The primer to be used in the practice of the invention is obtained by enzymatic digestion of a double-stranded DNA. In the PCR method which is currently applied, chemical synthesis of primers is essential. Therefore, the range of application of said method is limited to fragments whose base sequences are known and, in addition, an expensive DNA synthesizer and reagents therefor are needed. The present invention has obviated the need therefor. According to the invention, once a cDNA has been synthesized using reverse transcriptase, for instance, a primer can be prepared using said cDNA itself as a starting material without waiting for the results of complicated sequencing, and gene amplification can immediately be conducted by the PCR method for the analysis of related genes. It is of course possible to use not only cDNAs but also all DNA fragments as starting materials for the primer preparation according to the invention. The primer according to the invention is obtained by removing the majority or whole of one chain of a double-stranded DNA by digestion with an exonuclease capable of digesting the double-stranded DNA fragment only in one direction. When the primer is to be used in the inverted PCR method, an exonuclease for digestion from the 5′ terminus is to be used. Suitable for this purpose is, for example, the bacteriophase λ-derived exonuclease (J. W. Little et al., 1967, J. Biol. Chem., 242: 672~678). The primer fragment may have a length of 10 bases to 400 bases or even longer and, for each length, the temperature and time conditions in the thermal cycler should be adjusted. The invention has made it possible for the first time to prepare a primer of 100 bases or longer. When such a long primer is used, annealing can be conducted at a higher temperature as compared with the prior art method and this makes it possible to select the annealing temperature within a broader range and carry out the PCR rapidly and easily with a much higher specificity as compared with the use of shorter synthetic primers. Furthermore, when a fragment retaining a double-stranded DNA overlap as obtained by adjusting the exonuclease reaction for primer preparation is used as the primer, the DNA chain resulting from the amplification of a circular template DNA is a linear DNA having the whole base sequence of said template. This means that a desired DNA fragment among the cleavage product chromosomal genes and the like can be selectively amplified without the need of subcloning. It is thus evident that the methodology provided by the present invention is very useful in gene analysis.

### Example 1

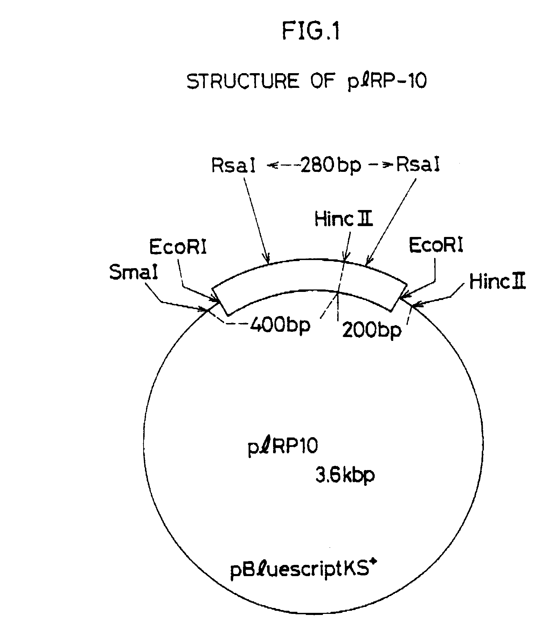

A primer DNA was prepared by λ exonuclease treatment. Thus, the cDNA for yale 10 kDa prolamin, a reserve protein in rice seeds, was obtained as reported previously (T. Masumura et al., 1989, Plant Mol. Biology, 12: 123-130). The plasmid pℓRP10 in which said cDNA has been cloned has a structure such as shown in Fig. 1. This plasmid is introduced into Escherichia coli and mass cultivation was performed with the resultant transformant. Cells were harvested from 1 liter of the culture and the plasmid DNA was recovered therefrom using the alkaline lysis technique and purified by ultracentrifugation in cesium chloride/ethidium bromide to give about 1 mg of the pℓRP10 plasmid DNA. A 50-µg portion of said plasmid DNA was digested with 50 units each of the restriction enzymes HincII and SmaI in a reaction buffer (33 mM Tris acetate, pH 7.9, 10 mM magnesium acetate, 0.5 mM DTT, 66 mM potassium acetate, 0.01% BSA) at 37°C for 2 hours. The reaction mixture was electrophoresed on a 3% agarose gel and two DNA fragments, 400 bp and 200 bp in length, were recovered from the corresponding bands of the gel by extraction followed by precipitation with ethanol. Of the DNA fragments thus obtained, 2 µg of the 400 bp fragment was treated in a buffer consisting of 67 mM glycine-KOH, pH 9.4, 2.5 mM MgCl₂ and 50 µg/ml BSA in the presence of 8 units of λ exonuclease at 37°C for 15 minutes. The reaction mixture was subjected to phenol/chloroform extraction. The subsequent precipitation with ethanol gave a primer DNA as a single-stranded DNA.

### Example 2

The rice genomic DNA was cleaved with a restriction enzyme, followed by self-ligation. A circular DNA thus obtained was amplified by the inverted PCR method. The rice DNA was prepared by the conventional method (Molecular Cloning, 269-294, Cold Spring Harbor Laboratory). A 10-µg portion of said DNA was treated for complete cleavage with 12 units of the restriction enzyme EcoRI in a high-salt-concentration reaction medium (50 mM Tris-C1, pH 7.5, 10 mM MgCl₂, 1 mM DTT,100 Mm NaCl) at 37°C for 2 hours. The reaction mixture was subjected to phenol/chloroform extraction and a DNA fragment was recovered by ethanol precipitation. This DNA was circularized by self-ligation using a Takara ligation kit, followed by phenol/chloroform extraction and ethanol precipitation. A circular genomic DNA for use as a template was thus obtained. Separately, a 280 bp fragment was derived from pℓRP10 by digestion with the restriction enzyme RsaI and a primer was prepared from this fragment in the same manner as in Example 1. To a reaction medium (10 mM Tris-Cl, pH 8.3, 1.5 mM MgCl₂, 0.01% (w/v) gelatin, 200 µM dNTPs, 0.5 U Ampli Tag DNA polymerase) was added 2 ng of the circular genomic DNA mentioned above, followed by addition of 200 ng of the primer prepared as described above. The reaction cycle was started and repeated 35 time. Each reaction cycle consisted of thermal denaturation (94°C, 1 minute), annealing (60°C, 2 minutes) and polymerase reaction (72°C, 2 minutes), which were conducted automatically and continuously in succession on a Cetus thermal cycler. In the final cycle, the reaction at 72°C was extended by 7 minutes.

The reaction mixture was subjected to phenol/chloroform extraction and ethanol precipitation. The DNA obtained was digested with the restriction enzyme RsaI and the digest was subjected to agarose gel electrophoresis. A band corresponding to the 280 bp cDNA fragment used as the starting material for primer preparation was observed. When this DNA was digested with the restriction enzyme EcoRI, a fragment of about 4,000 bp alone was detected. In Southern blotting using the prolamin cDNA as the probe, the DNA amplified in the above manner reacted with the probe. These results indicate that the genomic DNA between the EcoRI sites, including the segment corresponding to the cDNA for the rice 10-kDa prolamin and certain segments respectively upstream and downstream therefrom, had been amplified specifically.

## Claims

1. A method of amplifying DNA in vitro by polymerase chain reaction comprising the step of treating a double-stranded DNA fragment with an exonuclease to produce a primer and then conducting polymerase chain reaction using the said primer.

2. A method as claimed in Claim 1, wherein the exonuclease is a 5' terminus acting exonuclease.

3. A method as claimed in Claim 1 or Claim 2, wherein the exonuclease is Bacteriophage λ exonuclease.

4. A method as claimed in Claim 2 or Claim 3, wherein the polymerase chain reaction is inverse polymerase chain reaction.

5. A method as claimed in any preceding claim, wherein the DNA to be amplified is a chromosomal gene.

6. A method of amplifying a chromosomal gene comprising treating a double-stranded DNA fragment with a 5' terminus acting exonuclease to produce a primer, cleaving a chromosomal gene with a restriction enzyme and subjecting the resulting fragment to a self-ligation to form a template and then conducting an inverse polymerase chain reaction using the said primer and template in order to amplify the chromosomal gene.

7. A method as claimed in Claim 6, wherein the exonuclease is Bacteriophage λ exonuclease.

## Patentansprüche

1. Verfahren zum Amplifizieren von DNA in vitro durch Polymerase-Kettenreaktion, bei dem man ein doppelsträngiges DNA-Fragment zur Bildung eines Primers mit einer Exonuklease behandelt und anschließend die Polymerase-Kettenreaktion unter Verwendung dieses Primers durchführt.

2. Verfahren nach Anspruch 1, bei dem die Exonuklease eine am 5'-Terminus wirkende Exonuklease ist.

3. Verfahren nach Anspruch 1 oder Anspruch 2, bei dem die Exonuklease die Exonuklease des Bakteriophagen λ ist.

4. Verfahren nach Anspruch 2 oder Anspruch 3, bei dem die Polymerase-Kettenreaktion eine umgekehrte Polymerase-Kettenreaktion ist.

5. Verfahren nach irgendeinem vorhergehenden Anspruch, bei dem die zu amplifizierende DNA ein chromosomales Gen ist.

6. Verfahren zum Amplifizieren eines chromosomalen Gens, bei dem man ein doppelsträngiges DNA-Fragment mit einer am 5'-Terminus wirkenden Exonuklease zur Bildung eines Primers behandelt, ein chromosomales Gen mit einem Restriktionsenzym spaltet und das resultierende Fragment zur Bildung eines Templates einer Selbstligation zuführt, und man anschließend eine umgekehrte Polymerase-Kettenreaktion unter Verwendung des Primers und des Templates durchführt, um das chromosomale Gen zu amplifizieren.

7. Verfahren nach Anspruch 6, bei dem die Exonuklease die Exonuklease des Bakteriophagen λ ist.

## Revendications

1. Procédé d'amplification in vitro d'ADN par réaction en chaîne par polymérase, comprenant l'étape consistant à traiter un fragment d'ADN à deux brins par une exonucléase pour produire une amorce, puis à conduire la réaction en chaîne par polymérase en utilisant ladite amorce.

2. Procédé selon la revendication 1, dans lequel l'exonucléase est une exonucléase agissant sur l'extrémité 5'.

3. Procédé selon la revendication 1 ou 2, dans lequel l'exonucléase est une exonucléase de bactériophage λ.

4. Procédé selon la revendication 2 ou 3, dans lequel la réaction en chaîne par polymérase est une réaction en chaîne par polymérase inverse.

5. Procédé selon l'une quelconque des revendications 1 à 4, dans lequel l'ADN à amplifier est un gène chromosomique.

6. Procédé d'amplification d'un gène chromosomique, comprenant les étapes consistant à traiter un fragment d'ADN à deux brins par une exonucléase agissant sur l'extrémité 5' pour produire une amorce, à cliver un gène chromosomique avec une enzyme de restriction et à soumettre le fragment résultant à une auto-ligature pour former une matrice, puis à conduire une réaction en chaîne par polymérase inverse en utilisant lesdites amorce et matrice afin d'amplifier le gène chromosomique.

7. Procédé selon la revendication 6, dans lequel l'exonucléase est une exonucléase de bactériophage λ.
